# EUROPEAN PATENT APPLICATION

(11) **EP 4 420 589 A1**
(43) Date of publication of application: **28.08.2024**
(21) Application number: 22883197.0
(22) Date of filing: 23.08.2022
(51) Int. Cl.: A61B 1/00, A61B 1/045

(54) **PROGRAM, INFORMATION PROCESSING METHOD, INFORMATION PROCESSING DEVICE, AND DIAGNOSIS ASSISTANCE SYSTEM**

(30) Priority: 18.10.2021 JP 2021170434
(71) Applicant: HOYA CORPORATION, Shinjuku-ku Tokyo 160-8347 (JP)
(72) Inventor: NISHIDE, Akihiko, Tokyo 160-8347 (JP); SUGAI, Junko, Tokyo 160-8347 (JP); AMANO, Satsuki, Tokyo 160-8347 (JP)
(74) Representative: Schaumburg und Partner Patentanwälte mbB
(86) International application number: PCT/JP2022/031644
(87) International publication number: WO 2023/067885

(57) **Abstract**

A program causes a computer to execute processing including: acquiring information regarding a respiratory phase of a subject; acquiring a three-dimensional medical image obtained by capturing an inside of a body of the subject in synchronization with the respiratory phase; acquiring an endoscopic image of the subject from a lung field endoscope; specifying a position of the lung field endoscope in the three-dimensional medical image synchronized with the respiratory phase; and outputting the specified position of the lung field endoscope in association with the three-dimensional medical image.

## Description

### Technical Field

The present technology relates to a program, an information processing method, an information processing device, and a diagnosis support system.

The present application claims priority based on Japanese Patent Application No. 2021-170434 filed on October 18, 2021, the entire contents of which are incorporated herein by reference.

### Background Art

An endoscope is a medical instrument to be inserted into a body cavity of a subject to enable observation and treatment of a desired site, and includes an image capturing unit incorporated in a distal tip of an insertion tube to be inserted into the body cavity, and an illumination device illuminating an image capturing field of view of the image capturing unit. Patent Literature 1 discloses, as an endoscope device that observes the inside of a bile duct, a pancreatic duct, or the like, a master scope having an insertion portion that is insertable up to a duodenum and a master-slave scope type endoscope device capable of observing and treating up to the bile duct or the pancreatic duct by inserting a slave scope into a forceps channel of the master scope.

### Citation List

### Patent Literature

Patent Literature 1: JP 4-354929 A

### Summary of Invention

### Technical Problem

However, in the endoscope device described in Literature 1, it is not considered to specify, when a lung (bronchus) is to be observed, a position of the endoscope device (lung field endoscope device) in the lung corresponding to a respiratory phase by the lung.

In one aspect, an object is to provide a program and the like capable of specifying, corresponding to a respiratory phase by a lung to be observed, a position of a lung field endoscope device in the lung.

### Solution to Problem

A program in an aspect of the present disclosure causes a computer to execute processing including: acquiring information regarding a respiratory phase of a subject; acquiring a three-dimensional medical image obtained by capturing an inside of a body of the subject in synchronization with the respiratory phase; acquiring an endoscopic image of the subject from a lung field endoscope; specifying a position of the lung field endoscope in the three-dimensional medical image synchronized with the respiratory phase; and outputting the specified position of the lung field endoscope in association with the three-dimensional medical image.

An information processing method in an aspect of the present disclosure causes a computer to execute processing including: acquiring information regarding a respiratory phase of a subject; acquiring a three-dimensional medical image obtained by capturing an inside of a body of the subject in synchronization with the respiratory phase; acquiring an endoscopic image of the subject from a lung field endoscope; specifying a position of the lung field endoscope in the three-dimensional medical image synchronized with the respiratory phase; and outputting the specified position of the lung field endoscope in association with the three-dimensional medical image.

An information processing device in an aspect of the present disclosure includes: a phase information acquisition unit that acquires information regarding a respiratory phase of a subject; a three-dimensional medical image acquisition unit that acquires a three-dimensional medical image obtained by capturing an inside of a body of the subject in synchronization with the respiratory phase; an endoscopic image acquisition unit that acquires an endoscopic image of the subject from a lung field endoscope; a position specification unit that specifies a position of the lung field endoscope in the three-dimensional medical image synchronized with the respiratory phase; and an output unit that outputs the specified position of the lung field endoscope in association with the three-dimensional medical image.

A diagnosis support system in an aspect of the present disclosure includes: a lung field endoscope; an automatic operation mechanism that automatically operates the lung field endoscope; and a control unit that controls the automatic operation mechanism, and the control unit acquires information regarding a respiratory phase of a subject, acquires a three-dimensional medical image obtained by capturing an inside of a body of the subject in synchronization with the respiratory phase, acquires an endoscopic image of the subject from the lung field endoscope, specifies a position of the lung field endoscope in the three-dimensional medical image synchronized with the respiratory phase, and outputs operation support information regarding an operation of the lung field endoscope to the automatic operation mechanism on the basis of the three-dimensional medical image in which the position of the lung field endoscope is specified.

### Advantageous Effects of Invention

According to the present disclosure, it is possible to provide a program and the like that specify, corresponding to a respiratory phase by a lung to be observed, a position of a lung field endoscope device in the lung.

### Brief Description of Drawings

Fig. 1 is a schematic diagram illustrating an outline of a diagnosis support system according to a first embodiment.
Fig. 2 is a block diagram illustrating a configuration example of a lung field endoscope included in the diagnosis support system.
Fig. 3 is a block diagram illustrating a configuration example of an information processing device included in the diagnosis support system.
Fig. 4 is an explanatory diagram exemplifying a data layout of an endoscopic image DB.
Fig. 5 is an explanatory diagram illustrating an insertion distance (a value of an S coordinate) of the lung field endoscope.
Fig. 6 is a functional block diagram exemplifying functional units included in a control unit of the information processing device.
Fig. 7 is a flowchart illustrating an example of a processing procedure performed by the control unit of the information processing device.
Fig. 8 is an explanatory diagram illustrating an aspect of an integrated image display screen.
Fig. 9 is a flowchart illustrating an example of a processing procedure performed by a control unit of an information processing device according to a second embodiment (real-time three-dimensional image).
Fig. 10 is a schematic diagram illustrating an outline of a diagnosis support system according to a third embodiment (automatic operation mechanism).
Fig. 11 is a functional block diagram exemplifying functional units included in a control unit of an information processing device.

### Description of Embodiments

### (First Embodiment)

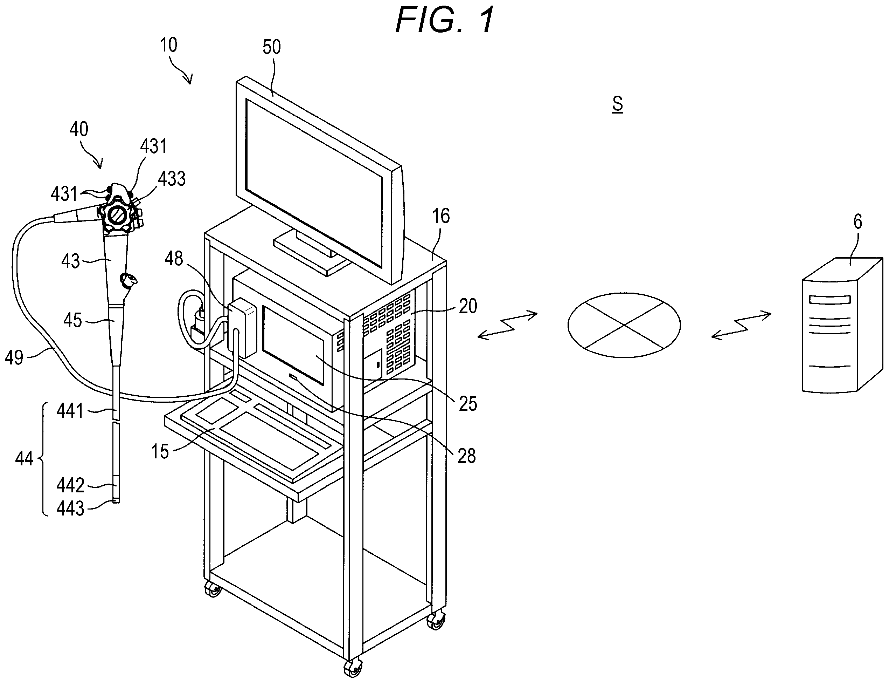

Hereinafter, the present invention will be specifically described with reference to the drawings illustrating embodiments of the present invention. Fig. 1 is a schematic diagram illustrating an outline of a diagnosis support system S according to a first embodiment. The diagnosis support system S includes an endoscope device 10 including a lung field endoscope 40 and an information processing device 6 communicably connected to the endoscope device 10, and outputs support information regarding diagnosis or the like of a lung (bronchus) of a subject.

The endoscope device 10 transmits an image (captured image) captured by an image sensor 445 of the lung field endoscope 40 to a processor 20 for an endoscope, and the processor 20 for an endoscope performs various types of image processing such as gamma correction, white balance correction, shading correction, and geometric correction for reducing image distortion, thereby generating an endoscopic image in the state of being easily visible to an operator. The endoscope device 10 outputs (transmits) the generated endoscopic image to the information processing device 6. The information processing device 6 that has acquired the endoscopic images transmitted from the endoscope device 10 performs various types of information processing on the basis of these endoscopic images, and outputs information regarding diagnosis support.

The endoscope device 10 includes the processor 20 for an endoscope, the lung field endoscope 40, and a display device 50. The display device 50 is, for example, a liquid crystal display device or an organic electro luminescence (EL) display device.

The display device 50 is installed on an upper stage of a storage shelf 16 with casters. The processor 20 for an endoscope is stored in a middle stage of the storage shelf 16. The storage shelf 16 is disposed in the vicinity of a bed for endoscopic examination (not illustrated). The storage shelf 16 includes a drawer type shelf on which a keyboard 15 connected to the processor 20 for an endoscope is mounted.

The processor 20 for an endoscope has a substantially rectangular parallelepiped shape and is provided with a touch panel 25 on one surface. A reading unit 28 is disposed at a lower portion of the touch panel 25. The reading unit 28 is, for example, a connection interface for performing reading and writing on a portable recording medium such as a USB connector, a secure digital (SD) card slot, or a compact disc read only memory (CD-ROM) drive.

The lung field endoscope 40 includes an insertion portion 44, an operation unit 43, a universal cord 49, and a scope connector 48. The operation unit 43 is provided with a control button 431. The insertion portion 44 is long and has one end connected to the operation unit 43 via a bend preventing portion 45. The insertion portion 44 has, in order from the operation unit 43 side, a soft portion 441, a bending section 442, and a distal tip 443. The bending section 442 bends according to an operation of a bending knob 433. Physical detection devices such as a three-axis acceleration sensor, a gyro sensor, a geomagnetic sensor, a magnetic coil sensor, and an endoscope insertion shape observation device (colonoscope navigation) may be implemented in the insertion portion 44, and detection results from these physical detection devices may be acquired when the lung field endoscope 40 is inserted into a body of a subject.

The universal cord 49 is long, and has a first end connected to the operation unit 43 and a second end connected to the scope connector 48. The universal cord 49 is soft. The scope connector 48 has a substantially rectangular parallelepiped shape.

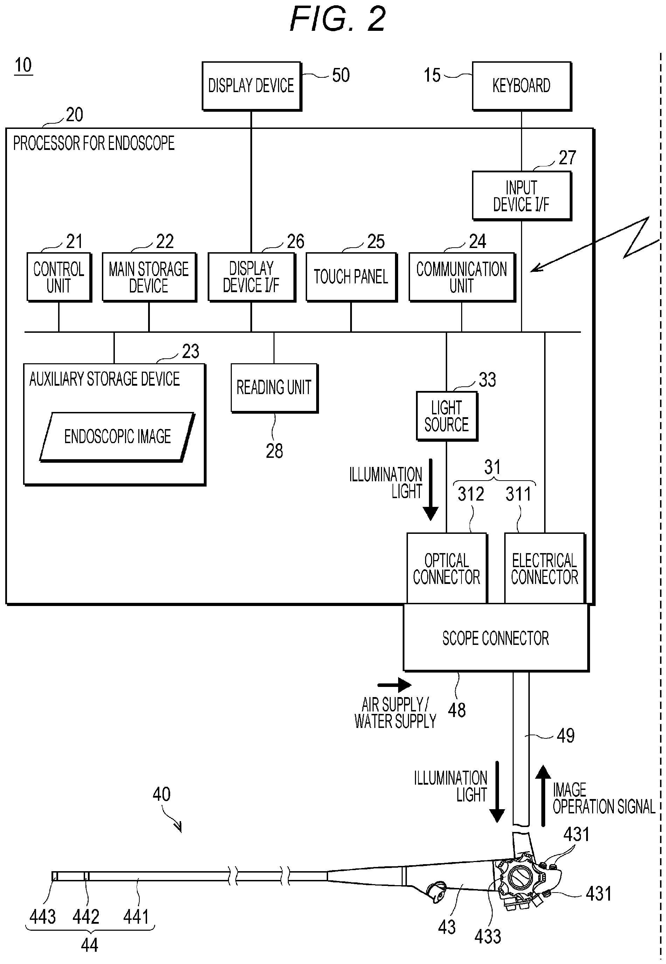

Fig. 2 is a block diagram illustrating a configuration example of the endoscope device 10 included in the diagnosis support system S. A control unit 21 is an arithmetic control device that executes a program of the present embodiment. One or a plurality of central processing units (CPUs), graphics processing units (GPUs), multi-core CPUs, or the like is used for the control unit 21. The control unit 21 is connected to each hardware unit constituting the processor 20 for an endoscope via a bus.

A main storage device 22 is, for example, a storage device such as a static random access memory (SRAM), a dynamic random access memory (DRAM), or a flash memory. The main storage device 22 temporarily stores information necessary during processing performed by the control unit 21 and a program being executed by the control unit 21. An auxiliary storage device 23 is, for example, a storage device such as an SRAM, a flash memory, or a hard disk and is a storage device with a larger capacity than that of the main storage device 22. The auxiliary storage device 23 may store, for example, an acquired captured image and a generated endoscopic image as intermediate data.

A communication unit 24 is a communication module or a communication interface for communicating with the information processing device 6 via a network in a wired or wireless manner and is, for example, a narrow-area wireless communication module such as Wi-Fi (registered trademark) or Bluetooth (registered trademark) or a wide-area wireless communication module such as 4G or LTE. The touch panel 25 includes a display unit such as a liquid crystal display panel and an input unit layered on the display unit. The communication unit 24 may communicate with an X-ray CT device 92, an MRI device, an ultrasonic diagnosis device, or a storage device (not illustrated) that stores data output from these devices.

A display device I/F 26 is an interface that connects the processor 20 for an endoscope and the display device 50 to each other. An input device I/F 27 is an interface that connects the processor 20 for an endoscope and an input device such as the keyboard 15 to each other.

A light source 33 is, for example, a high-luminance white light source such as a white LED or a xenon lamp. The light source 33 is connected to the bus via a driver (not illustrated). The control unit 21 controls switching the light source 33 on and off and changing luminance. Illumination light emitted from the light source 33 is incident on an optical connector 312. The optical connector 312 engages with the scope connector 48 to supply the illumination light to the lung field endoscope 40.

An outline of functions of the lung field endoscope 40 connected to the processor 20 for an endoscope will be described. A fiber bundle, a cable bundle, an air supply tube, and the like are inserted inside the scope connector 48, the universal cord 49, the operation unit 43, and the insertion portion 44. The illumination light emitted from the light source 33 is emitted from an illumination window provided at the distal tip 443 via the optical connector 312 and the fiber bundle. An image sensor provided at the distal tip 443 captures a range illuminated by the illumination light. A captured image is transmitted from the image sensor to the processor 20 for an endoscope via the cable bundle and an electrical connector 311. The light source 33 may be an LED provided at the distal tip 443, and in this case, the optical connector 312 and the fiber bundle are unnecessary.

The control unit 21 of the processor 20 for an endoscope functions as an image processing unit 211 by executing a program stored in the main storage device 22. The image processing unit 211 performs various types of image processing such as gamma correction, white balance correction, shading correction, and geometric correction for reducing image distortion on an image (captured image) output from the lung field endoscope 40, and outputs the image as an endoscopic image.

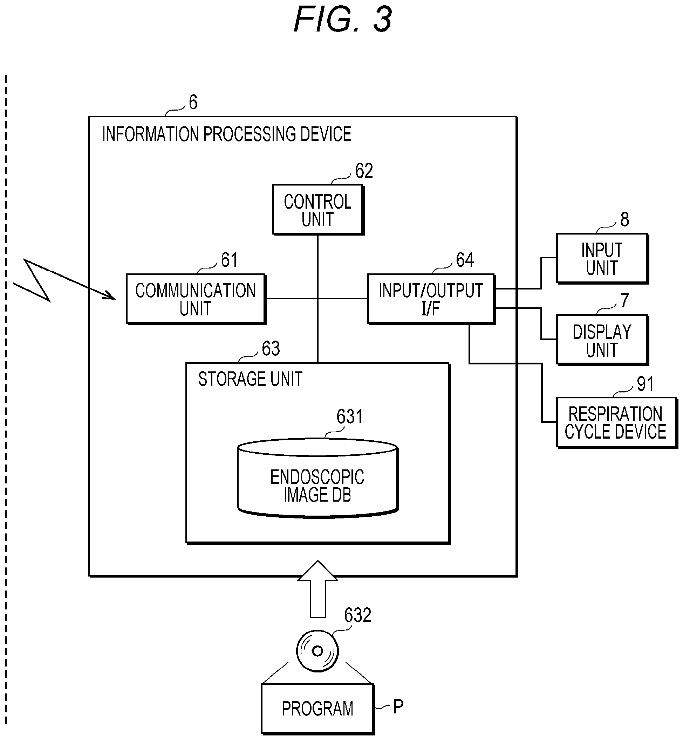

Fig. 3 is a block diagram illustrating a configuration example of the information processing device 6 included in the diagnosis support system S. The information processing device 6 includes a control unit 62, a communication unit 61, a storage unit 63, and an input/output I/F 64. The information processing device 6 is, for example, a server device or a personal computer. The server device not only includes a single server device, but also a cloud server device configured by a plurality of computers, or a virtual server device. The information processing device 6 may be provided as a cloud server positioned on an external network accessible from the processor 20 for an endoscope.

The control unit 62 includes one or a plurality of arithmetic processing devices having a time counting function, such as central processing units (CPUs), micro-processing units (MPUs), and graphics processing units (GPUs), and performs various types of information processing, control processing, and the like related to the information processing device 6 by reading and executing a program P stored in the storage unit 63. Alternatively, the control unit 62 may include a quantum computer chip, and the information processing device 6 may be a quantum computer.

The storage unit 63 includes a volatile storage area such as a static random access memory (SRAM), a dynamic random access memory (DRAM), or a flash memory, and a nonvolatile storage area such as an EEPROM or a hard disk. The storage unit 63 stores the program P (program product) and data to be referred to at the time of processing in advance. The program P (program product) stored in the storage unit 63 may be the program P (program product) which is stored by being read from a recording medium 632 readable by the information processing device 6. In addition, the program P may be downloaded from an external computer (not illustrated) connected to a communication network (not illustrated) and is stored in the storage unit 63. The storage unit 63 stores an endoscopic image database (DB) 631 to be described later. In addition, the storage unit 63 may store entity files (instance files of a neural network (NN)) constituting various learning models to be described later.

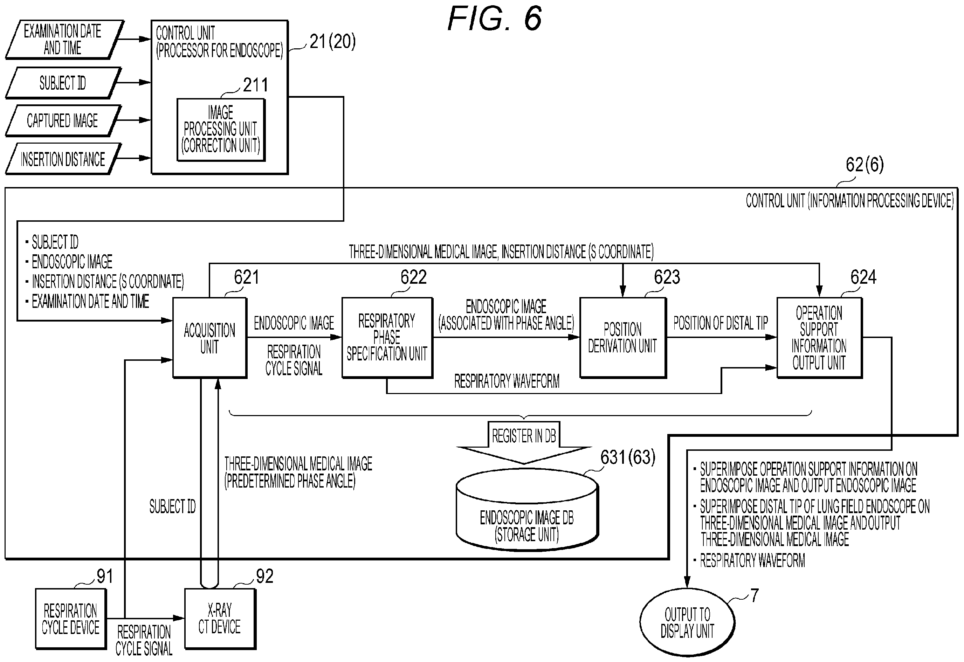

The communication unit 61 is a communication module or a communication interface for communicating with the endoscope device 10 in a wired or wireless manner and is, for example, a narrow-area wireless communication module such as Wi-Fi (registered trademark) or Bluetooth (registered trademark) or a wide-area wireless communication module such as 4G or 5G. The communication unit 61 may communicate with the X-ray CT device 92, an MRI device (see Fig. 6), an ultrasonic diagnosis device, or a storage device (not illustrated) that stores data output from these devices.

The input/output I/F 64 is a communication interface that conforms to, for example, a communication standard such as USB or DSUB, and that is for serially communicating with an external device connected to the input/output I/F 64. For example, a display unit 7 such as a display, and an input unit 8 such as a keyboard are connected to the input/output I/F 64, and the control unit 62 outputs, to the display unit 7, a result of information processing performed based on an execution command or an event input from the input unit 8. A respiration synchronization device 91 (see Fig. 6) may be connected to the input/output I/F 64.

The respiration synchronization device 91 is a device that includes a marker or the like attached to an abdomen or the like of a subject and outputs a signal (respiration synchronization signal) corresponding to a cycle of respiration (respiration cycle). By receiving the respiration synchronization signal from the respiration synchronization device 91 via the input/output I/F 64, the information processing device 6 can acquire information regarding a state of respiration of the subject such as a phase in the respiration cycle, a time point such as a maximum inspiration state (100% state) or a maximum expiration state (0% state), and a respiratory waveform.

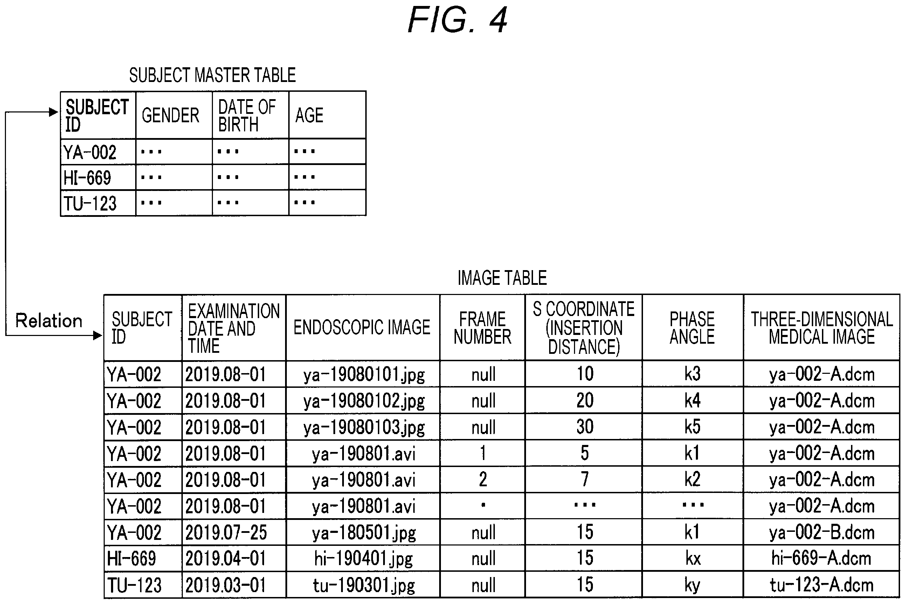

Fig. 4 is an explanatory diagram exemplifying a data layout of the endoscopic image DB 631. The endoscopic image DB 631 is stored in the storage unit 63 of the information processing device 6, and is configured by database management software such as a relational database management system (RDBMS) implemented in the information processing device 6. Alternatively, the endoscopic image DB 631 may be stored in a predetermined storage area accessible from the information processing device 6, such as a storage device communicably connected to the information processing device 6. Alternatively, the endoscopic image DB 631 may be stored in the main storage device 22 of the endoscope device 10. That is, the predetermined storage area includes the storage unit 63 of the information processing device 6, the main storage device 22 of the endoscope device 10, and a storage device accessible from the information processing device 6 or the endoscope device 10. The information processing device 6 may acquire an endoscopic image, an examination date and time, and attribute information of a subject output by the processor 20 for an endoscope, and register the acquired endoscopic image, examination date and time, and attribute information of the subject in an examination result DB 631. Alternatively, the endoscopic image, the examination date and time, and the attribute information of the subject directly output from the processor 20 for an endoscope may be directly registered in the examination result DB 631.

The endoscopic image DB 631 includes, for example, a subject master table and an image table, and the subject master table and the image table are set to be associated with each other by a subject ID that is an item (metadata) included in both the tables.

The subject master table includes, for example, the subject ID, a gender, a date of birth, and an age as management items (metadata). In the item (field) of the subject ID, ID information is stored in order to uniquely specify the subject who has undergone an endoscopic examination. In the items (fields) of the gender and the date of birth, biological attributes including the gender and the date of birth corresponding to the subject ID are stored. In the item (field) of the age, the age at a current time point calculated based on the date of birth is stored. The gender and the age are managed as biological information of the subject by the subject master table.

The image table includes, for example, the subject ID, an examination date and time, an endoscopic image, a frame number, an S coordinate (insertion distance), a phase angle, and a three-dimensional medical image, as management items (metadata).

In the item (field) of the subject ID, a value of an ID of each subject that is for associating with the biological attributes of the subject managed in the subject master table is stored. In the item (field) of the examination date and time, a date and time when the subject corresponding to the subject ID has undergone the endoscopic examination is stored. In the item (field) of the endoscopic image, the endoscopic image corresponding to the subject ID is stored as object data. The endoscopic image may be a still image in, for example, a jpeg format with one frame or a moving image in, for example, an avi format with several frames. In the item (field) of the endoscopic image, information indicating a storage location (file path) of the endoscopic image stored as a file may be stored.

In the item (field) of the frame number, in a case where the endoscopic image is a moving image, a frame number of the moving image is stored. Even in a case where the endoscopic image is a moving image, by storing the frame number of the moving image, the moving image can be handled in the same manner as a still image, and can be associated with position information (coordinates in an in-vivo coordinate system) of the three-dimensional medical image to be described later.

In the item (field) of the S coordinate (insertion distance), an insertion distance of the lung field endoscope 40 at a capturing time point of the endoscopic image stored in the same record is stored as a value of the S coordinate. Deriving of the insertion distance (S coordinate) or the like will be described later.

In the item (field) of the phase angle, a phase angle (phase angle in a respiratory phase) at a capturing time point of the endoscopic image stored in the same record is stored. Although details will be described later, the information processing device 6 (control unit 62) acquires a respiration synchronization signal from the respiration synchronization device 91 in parallel with acquisition of the endoscopic image, and specifies the phase angle in the respiratory phase (respiration cycle) on the basis of the acquired respiration synchronization signal. The information processing device 6 (control unit 62) stores the specified phase angle in the item (field) of the phase angle, whereby each endoscopic image and the phase angle are stored in association with each other.

In the item (field) of the three-dimensional medical image, for example, a three-dimensional medical image in a digital imaging and communications in medicine (DICOM) format, which is generated on the basis of data output from means capable of capturing a three-dimensional image of inside of a body, such as a the X-ray CT device 92 (X-ray CT, X-ray cone beam CT), an MRI device (MRI-CT), or an ultrasonic diagnosis device, is stored as object data. Alternatively, information indicating a storage location (file path) of the three-dimensional medical image stored as a file may be stored.

The image table may further include items (fields) of a bending history, a viewpoint position, and a viewpoint direction. In this case, in the item (field) of the bending history, for example, bending history information detected using an endoscope insertion shape detection device connected to the processor 20 for an endoscope is stored. In the item (field) of the viewpoint position, coordinates of the lung field endoscope 40 in the body at a time point when the endoscopic image is captured, that is, coordinates of the three-dimensional medical image in the coordinate system (in-vivo coordinate system) are stored. In the item (field) of the viewpoint direction, an orientation of the lung field endoscope 40 at the time point when the endoscopic image is captured, that is, a rotation angle in the coordinate system (coordinates in the in-vivo coordinate system) of the three-dimensional medical image is stored.

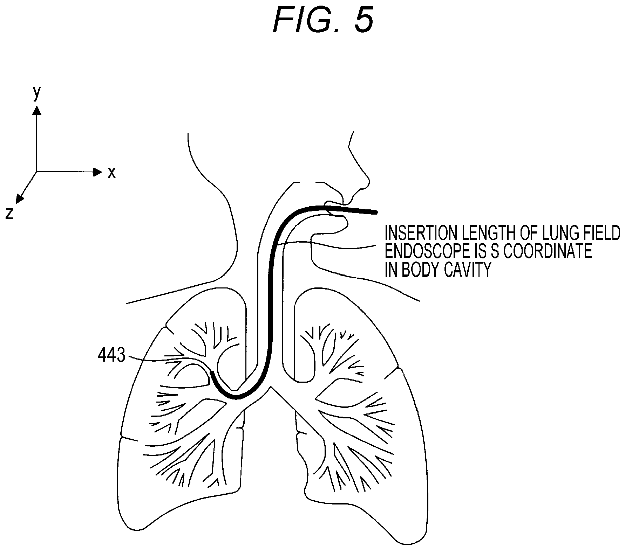

Fig. 5 is an explanatory diagram illustrating an insertion distance (value of the S coordinate) of the lung field endoscope 40. As illustrated in the drawing, a bronchus of a lung or the like captured by the lung field endoscope 40 is represented by a three-dimensional shape in a three-dimensional medical image. A space is formed inside an inner wall of the bronchus or the like, and the space serves as an insertion path through which the lung field endoscope 40 is inserted. The S coordinate, which is the insertion distance of the lung field endoscope 40, corresponds to a location which is inside the insertion path (inside the inner wall of the bronchus or the like) and in which a path length of the insertion path is substantially equal to the insertion distance. Thus, coordinates of the distal tip 443 of the lung field endoscope 40 positioned inside the inner wall of the bronchus or the like can be derived on the basis of the S coordinate. The insertion distance is obtained by, for example, providing a measurement unit including a roller encoder or the like on a mouthpiece disposed at a mouth of a subject and measuring a distance by which the lung field endoscope 40 is inserted into a body. A position where the measurement unit is provided is not limited to the mouthpiece, and a measurement unit using a non-contact sensor may be disposed in a throat of the subject. The S coordinate may be further corrected using the bending history information.

Fig. 6 is a functional block diagram exemplifying functional units included in the control unit 62 of the information processing device 6. The control unit 21 of the processor 20 for an endoscope (endoscope device 10) executes a program stored in the main storage device 22, thereby functioning as the image processing unit 211. the control unit 62 of the information processing device 6 executes the program P stored in the storage unit 63, thereby functioning as an acquisition unit 621, a respiratory phase specification unit 622, a position derivation unit 623, and an operation support information output unit 624.

The image processing unit 211 of the processor 20 for an endoscope performs various types of image processing such as gamma correction, white balance correction, and shading correction on an image (captured image) output from the lung field endoscope 40, and outputs the image as an endoscopic image. The image processing unit 211 outputs (transmits), to the information processing device 6, the generated endoscopic image and an examination date and time based on a capturing time point of the endoscopic image. The image processing unit 211 may further output a subject ID input from the keyboard 15 to the information processing device 6. The image processing unit 211 may output, to the information processing device 6, information regarding an insertion distance (S coordinate) of the lung field endoscope 40 that is output from a sensor disposed in the insertion portion 44 (flexible tube) of the lung field endoscope 40 in order to measure a surrounding environment of the lung field endoscope 40. The image processing unit 211 may, for example, superimpose the information regarding the insertion distance of the lung field endoscope 40 acquired from the sensor on the endoscopic image, and display the superimposed image on the display device.

Examples of the sensor for acquiring the S coordinate, which is the distance by which the lung field endoscope 40 is inserted into a body, include a temperature sensor, an optical sensor, a pressure sensor, a wetting sensor (electrode), and a humidity sensor. For example, in a case where the sensor is an optical sensor, the optical sensor is disposed inside the insertion portion 44 (flexible tube). However, the optical sensor can receive light even when the insertion portion 44 (flexible tube) is inserted into the body. Therefore, it is possible to determine that a portion where the optical sensor receives more light is outside of the body and a portion where the optical sensor receives less light is inside of the body. Then, the control unit 21 of the processor 20 for an endoscope can derive the S coordinate, which is the distance (length) by which the insertion portion 44 (flexible tube) is inserted into the body, by specifying the optical sensor that is located at a boundary position which is a body cavity insertion site on the basis of a signal obtained by the optical sensor.

The control unit 21 of the processor 20 for an endoscope acquires bending history information of the lung field endoscope 40 inserted into the body, and determines an insertion situation of the lung field endoscope 40 according to the acquired bending history information. The control unit 21 of the processor 20 for an endoscope may detect the bending history information using, for example, an endoscope insertion shape detection device (not illustrated) connected to the processor 20 for an endoscope. For example, as disclosed in JP 2019-37643 A, the endoscope insertion shape detection device may be a device in which a plurality of magnetic coils is disposed inside the insertion portion 44 of the lung field endoscope 40 at predetermined intervals along a longitudinal direction of the insertion portion 44. The bending history information indicates a physical parameter or information regarding bending, such as a bending angle or a bending direction.

The acquisition unit 621 acquires the subject ID, the examination date and time, the endoscopic image, the S coordinate (insertion distance), and the bending history information output by the processor 20 for an endoscope. Based on the acquired subject ID, the acquisition unit 621 acquires a three-dimensional medical image of the subject that is output from means capable of capturing a three-dimensional image of the inside of the body, such as the X-ray CT device 92, a cone beam CT device, an MRI device, or an ultrasonic diagnosis device, which is communicably connected. In a case where a three-dimensional medical image, which is output from another examination device capable of capturing a three-dimensional image of the inside of the body, such as the X-ray CT device 92, a cone beam CT device, an MRI device, or an ultrasonic diagnosis device, is already stored in, for example, an external server (not illustrated), the information processing device 6 may access the external server, and acquire the three-dimensional medical image of the subject based on the subject ID output from the processor 20 for an endoscope.

The three-dimensional medical image is, for example, an image represented by volume data including tomographic image data, which is output from means capable of capturing a three-dimensional image of the inside of the body, such as the X-ray CT device 92, a cone beam CT device, an MRI device, or an ultrasonic diagnosis device, or is an image represented by volume data which is output from a Multi Slice X-ray CT device and an X-ray cone beam CT device using an X-ray flat panel. In a case where the X-ray CT device 92 or the cone beam CT device is used, for example, dual energy imaging may be performed by the X-ray CT, and an image in which a composition (body composition) of each pixel of the three-dimensional medical image can be identified by an effective mass number (effective-Z) may be used. In a case where the MRI device is used, an image obtained by adding information regarding a composition (body composition) of each pixel of the three-dimensional medical image, such as fat or lactic acid, may be used.

The acquired three-dimensional medical image is an image reconstructed according to a respiratory phase, and is, for example, an image generated based on a CT data group captured at a time point of a predetermined phase angle (2π) corresponding to normal end expiration (when breathing out: 0% state). Therefore, since the three-dimensional medical image corresponding to the respiratory phase is, for example, an image indicating a state of a lung in the normal end expiration (phase angle: 2π), it is possible to reduce an influence of body motion due to respiration by the lung to be examined.

The three-dimensional medical image is an image reconstructed (generated) by examination of the X-ray CT device 92 performed before examination by the lung field endoscope 40, and in a case where there is a region of interest (RI) such as a tumor in the lung to be examined, a position of the region of interest is specified in an in-vivo coordinate system by the three-dimensional medical image. In a case where the three-dimensional medical image is input when presence or absence of the region of interest such as a tumor is specified on the basis of the three-dimensional medical image, a region of interest model (learning model) that outputs the position and a type of the region of interest included in the three-dimensional medical image may be used. The region of interest model may be configured by, for example, a neural network or the like having a segmentation function such as R-CNN or YOLO.

The acquisition unit 621 further acquires, from the respiration synchronization device 91, a signal (respiration synchronization signal) corresponding to a cycle of respiration (respiration cycle). Output of the respiration synchronization signal by the respiration synchronization device 91, that is, detection of the respiratory phase and the acquisition (capturing) of the endoscopic image are performed simultaneously in parallel. The acquisition unit 621 outputs the acquired respiration synchronization signal and the endoscopic image to the respiratory phase specification unit 622 in association with each other.

The respiratory phase specification unit 622 specifies a phase angle in the respiratory phase at the time point (capturing time point) when the endoscopic image is captured according to the respiration synchronization signal and the endoscopic image output from the acquisition unit 621. The respiratory phase specification unit 622 may generate a respiratory waveform having periodicity on the basis of the acquired respiration synchronization signal. The respiratory phase specification unit 622 specifies the phase angle in the respiratory phase (the phase angle at the capturing time point of the endoscopic image) on the basis of the acquired respiration synchronization signal, and determines whether or not the phase angle of the endoscopic image captured (acquired) at the current time point matches the phase angle of the three-dimensional medical image. The respiratory phase specification unit 622 may store, in the endoscopic image DB 631 stored in the storage unit 63, the phase angle specified on the basis of the respiration synchronization signal and the endoscopic image captured at the time point when the respiration synchronization signal is acquired in association with each other. By storing, in this way, each of the captured endoscopic images (each frame in a case where the endoscopic image is a moving image) in association with the phase angle in the respiratory phase at the time point when the endoscopic image is captured, various types of operations or processing synchronized with the respiratory phase (respiration cycle) can be performed on the endoscopic image.

The respiration synchronization signal and the endoscopic image that are acquired by the acquisition unit 621 correspond to each other on a time axis. As a result, the phase angle in the respiratory phase at the time point (capturing time point) when the endoscopic image is acquired is specified by the respiration synchronization signal acquired (detected) at the time point (capturing time point). Therefore, the respiration synchronization signal corresponds to a synchronization signal that synchronizes each of the endoscopic images continuously acquired with the respiratory phase (respiration cycle). In a case where the endoscopic image is a moving image, it goes without saying that the acquisition time point of the endoscopic image is an acquisition time point of a frame (still image) constituting the moving image. In this way, by acquiring the respiration synchronization signal from the respiration synchronization device 91 in parallel (simultaneously) with the examination (capturing of the endoscopic image) by the lung field endoscope 40, it is possible to associate the phase angle in the respiratory phase at the capturing time point of the endoscopic image with each of the endoscopic images acquired in time series, that is, continuously over time. The three-dimensional medical image is reconstructed (generated) on the basis of a predetermined phase angle (for example, the phase angle corresponding to the normal end expiration: 2n). Therefore, by using the endoscopic image with which the same phase angle as the phase angle of the three-dimensional medical image is associated, it is possible to reduce an influence of body motion due to respiration and to accurately specify a position of the lung field endoscope 40 (distal tip 443) in the three-dimensional medical image (in-vivo coordinate system).

The position derivation unit 623 derives the position of the distal tip 443 of the lung field endoscope 40 in the three-dimensional medical image (in-vivo coordinate system) having the predetermined phase angle in the respiratory phase on the basis of the insertion distance (S coordinate), a bending history, and the like of the lung field endoscope 40 output from the acquisition unit 621. When the position of the distal tip 443 of the lung field endoscope 40 is derived, the position derivation unit 623 may derive the position of the lung field endoscope 40 (distal tip 443) using the insertion distance (S coordinate) and the like at the capturing time point of the endoscopic image having the same phase angle as the phase angle of the three-dimensional medical image. The S coordinate may be further corrected using the bending history information.

The fact that the phase angle of the three-dimensional medical image is the same as the phase angle at the capturing time point of the endoscopic image is not limited to a case where these phase angles completely match, and it is intended that the phase angle at the capturing time point of the endoscopic image is included in an angle range allowed when a doctor or the like operates the lung field endoscope 40, such as a case where, for example, the phase angle at the capturing time point of the endoscopic image falls within a predetermined angle range such as ±5° relative to the phase angle of the three-dimensional medical image.

The operation support information output unit 624 outputs, as operation support information, the position of the distal tip 443 of the lung field endoscope 40 and information regarding a path from the position of the distal tip 443 to the position of the region of interest such as a lesion. The operation support information output unit 624 may output and display the position of the lung field endoscope 40 (distal tip 443) and the information regarding the path to the region of interest in association with each other by, for example, superimposing or adding these on or to the three-dimensional medical image and the endoscopic image.

When the operation support information output unit 624 outputs the three-dimensional medical image by superimposing the lung field endoscope 40 (distal tip 443), in a case where the phase angles of the endoscopic image and the three-dimensional medical image match, the position of the lung field endoscope 40 (distal tip 443) superimposed and displayed on the three-dimensional medical image is not affected by body motion due to respiration and has high suitability. In a case where the phase angles of the endoscopic image and the three-dimensional medical image do not match, it is considered that the position of the lung field endoscope 40 (distal tip 443) superimposed and displayed on the three-dimensional medical image is affected by body motion due to respiration, and it is highly likely that the position is different from an actual position. The operation support information output unit 624 may change a display form of the lung field endoscope 40 (distal tip 443) superimposed and displayed on the endoscopic image or the three-dimensional medical image depending on whether the phase angles of the endoscopic image and the three-dimensional medical image match or do not match. For example, in a case where the phase angles of the endoscopic image and the three-dimensional medical image match, the lung field endoscope 40 (distal tip 443) may be displayed in blue, and in a case where the phase angles do not match, the lung field endoscope 40 (distal tip 443) may be displayed in yellow. Each functional unit such as the operation support information output unit 624 may store, in the endoscopic image DB 631, various types of data to be acquired or output.

In the present embodiment and the like, the processing of each functional unit has been described by being divided into the processing performed by the control unit 21 of the processor 20 for an endoscope and the processing performed by the control unit 62 of the information processing device 6. However, the division into these types of processing is an example, and the present invention is not limited hereto. The control unit 21 of the processor 20 for an endoscope may function as all the functional units performed by the control unit 62 of the information processing device 6. Alternatively, the control unit 21 of the processor 20 for an endoscope and the control unit 62 of the information processing device 6 may function in cooperation as each functional unit in the series of processing by performing, for example, interprocess communication.

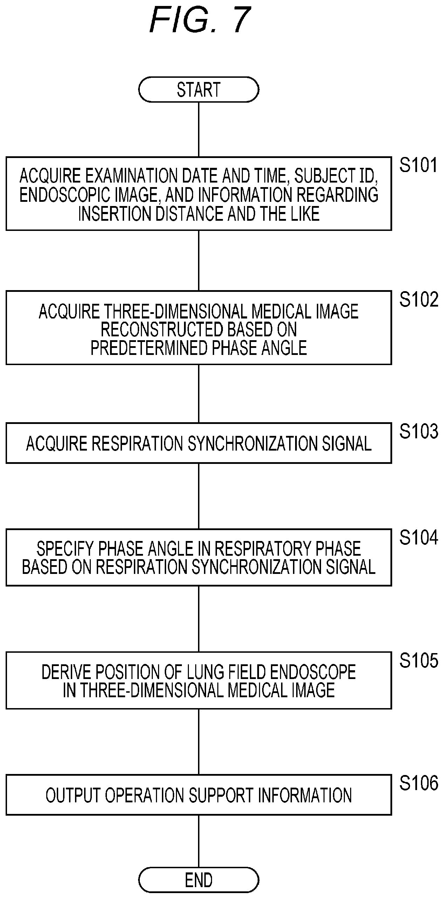

Fig. 7 is a flowchart illustrating an example of a processing procedure performed by the control unit 62 of the information processing device 6. The control unit 62 of the information processing device 6 starts processing of the flowchart on the basis of, for example, contents input from the input unit 8 connected to the device.

The control unit 62 of the information processing device 6 acquires an examination date and time, a subject ID, an endoscopic image, and information regarding an insertion distance and the like, which are output from the processor 20 for an endoscope (S101). The control unit 62 of the information processing device 6 may further acquire bending history information of the lung field endoscope 40 inserted into a body from the processor 20 for an endoscope in addition to the insertion distance (S coordinate).

The control unit 62 of the information processing device 6 acquires, from the X-ray CT device 92 or the like, a three-dimensional medical image reconstructed on the basis of a predetermined phase angle (S102). The control unit 62 of the information processing device 6 acquires, from the X-ray CT device 92 or the like, the three-dimensional medical image reconstructed on the basis of the predetermined phase angle, that is, the three-dimensional medical image synchronized with a respiratory phase. The three-dimensional medical image is an image reconstructed on the basis of a CT image (tomographic image data) based on a result of examination by the X-ray CT device 92 or the like performed on the same subject before examination of a lung (bronchus) (capturing of the endoscopic image) by the lung field endoscope 40. Using the acquired subject ID, the control unit 62 of the information processing device 6 accesses the X-ray CT device 92, a data management server of the three-dimensional medical image, or the like, and acquires the three-dimensional medical image that is a result of examination by the X-ray CT device 92 or the like previously taken by the subject of the lung field endoscope 40.

In the examination of the lung by the X-ray CT device 92, body motion included in the captured CT image (tomographic image data) is changed by body motion due to respiration in the lung, and the three-dimensional medical image (X-ray CT three-dimensional image) in the present embodiment is the three-dimensional medical image reconstructed on the basis of the predetermined phase angle (three-dimensional medical image synchronized with the respiratory phase). That is, in parallel with the examination (capturing of the CT image) of the lung by the X-ray CT device 92, a respiratory waveform (respiratory phase) is measured on the basis of a respiration synchronization signal acquired from the respiration synchronization device 91, and the CT images (tomographic image data) having a predetermined respiratory phase are aggregated and rearranged, thereby reconstructing the three-dimensional medical image in the predetermined respiratory phase. By using the three-dimensional medical image reconstructed on the basis of the predetermined phase angle (for example, the phase angle corresponding to the normal end expiration: 2n) in this way, it is possible to reduce an influence of body motion due to respiration.

The three-dimensional medical image is not limited to the case of being reconstructed by a single respiratory phase (phase angle), and may be reconstructed on the basis of a plurality of respiratory phases (phase angles). For example, for the CT image (tomographic image data) captured by the X-ray CT device 92, two sets (two phases) of three-dimensional medical images may be reconstructed in each of two respiratory phases in a maximum inspiration state (100% state: π) and a maximum expiration state (0% state: 2π) in one respiration cycle. The number of phases of the respiratory phase may be equal to or greater than two, and for example, a processing unit angle may be n/6, and 12 sets (12 phases) of three-dimensional medical images may be reconstructed. The three-dimensional medical images reconstructed in a plurality of phases in this way are segmented in a plurality of phase units, and may be configured as an archive file of a three-dimensional medical image group in which individual phase angles are associated. The position of the lung field endoscope is specified for each of the segmented three-dimensional medical images.

As described above, the three-dimensional medical image is captured before the examination by the lung field endoscope 40, and the three-dimensional medical image includes the entire lung of the subject. Therefore, in a case where there is a region of interest (ROI) such as a lesion such as a tumor or a lesion candidate in the lung, the region of interest is included in the three-dimensional medical image. With respect to the region of interest included in the three-dimensional medical image in this way, a position of the region of interest (coordinates of the in-vivo coordinate system) in the three-dimensional medical image is specified (added to the three-dimensional medical image) by diagnosis of a doctor or the like, for example. The region of interest may be specified using a learning model (region of interest model) that outputs a region and a type of the region of interest in a case where the three-dimensional medical image is input.

The control unit 62 of the information processing device 6 acquires, from the respiration synchronization device 91, a signal (respiration synchronization signal) corresponding to a cycle of respiration (respiration cycle) (S103). The control unit 62 of the information processing device 6 specifies a phase angle in the respiratory phase on the basis of the respiration synchronization signal (S104). The respiration synchronization device 91 includes a marker or the like attached to an abdomen or the like of the subject of the lung field endoscope 40, and outputs the signal (respiration synchronization signal) corresponding to the cycle of respiration (respiration cycle). The control unit 62 of the information processing device 6 acquires the respiration synchronization signal from the respiration synchronization device 91 in parallel with the acquisition of the endoscopic image, and specifies the phase angle in the respiratory phase on the basis of the acquired respiration synchronization signal. Alternatively, a device that measures body motion from a video image (moving image) obtained by capturing the subject and obtains the respiratory phase may be used.

Since the capturing (acquisition) of the endoscopic image and the specification of the phase angle by the respiration synchronization signal are continuously performed in parallel, the control unit 62 of the information processing device 6 can continuously specify the phase angle at the capturing time point of the endoscopic image. The control unit 62 of the information processing device 6 may store, in the storage unit 63 (store, in the endoscopic image DB 631), the specified phase angle in association with the endoscopic image. In a case where the endoscopic image is a moving image, a phase angle at a time point when a frame (still image) constituting the moving image is captured may be stored in association with the frame (still image). In this way, by associating the phase angle at the time point when the endoscopic image is captured with each endoscopic image, synchronization between the endoscopic image and the respiratory phase can be achieved. Furthermore, since the insertion distance (S coordinate) and a bending history at the capturing time point of the endoscopic image are associated with the endoscopic image, the respiratory phase is also associated with the insertion distance (S coordinate) and the bending history.

The control unit 62 of the information processing device 6 derives the position of the lung field endoscope 40 in the three-dimensional medical image (S105). The three-dimensional medical image corresponds to the predetermined phase angle, and the control unit 62 of the information processing device 6 extracts the endoscopic image having the same phase angle as the phase angle of the three-dimensional medical image. As a result, the acquired endoscopic image and three-dimensional medical image can be synchronized on the basis of the phase angle (associated by the same phase angle). The fact that the phase angle of the three-dimensional medical image is the same as the phase angle at the capturing time point of the endoscopic image is not limited to a case where these phase angles completely match, and it may be a case where, for example, the phase angle at the capturing time point of the endoscopic image falls within a predetermined angle range such as ±5° relative to the phase angle of the three-dimensional medical image. Since the extracted endoscopic image and the three-dimensional medical image have the same phase angle, an influence of body motion due to respiration between these images is small, and it is possible to improve accuracy of deriving the position of the lung field endoscope 40 (distal tip 443) in the three-dimensional medical image.

The control unit 62 of the information processing device 6 specifies the position of the distal tip 443 of the lung field endoscope 40 in the three-dimensional medical image (in-vivo coordinate system) on the basis of the insertion distance (S coordinate) and the bending history of the extracted endoscopic image (endoscopic image having the same phase angle as that of the three-dimensional medical image) at the capturing time point. By using the insertion distance (S coordinate) and the bending history of the lung field endoscope 40, it is possible to specify a path length of an insertion path (bronchus of the lung or the like) into which the lung field endoscope 40 is inserted and an insertion direction, and as a result, it is possible to derive each of coordinates of the distal tip 443 of the lung field endoscope 40 positioned inside an inner wall of the bronchus or the like. When the position of the distal tip 443 of the lung field endoscope 40 in the three-dimensional medical image (in-vivo coordinate system) is derived on the basis of the insertion distance (S coordinate) and the like of the lung field endoscope 40, the control unit 62 of the information processing device 6 may use a virtual endoscopic image as disclosed in, for example, International Publication No. WO 2021/054360.

The control unit 62 of the information processing device 6 outputs operation support information on the basis of the derived position of the lung field endoscope 40 and the position of the region of interest included in the three-dimensional medical image (S106). The control unit 62 of the information processing device 6 specifies, in the three-dimensional medical image (in-vivo coordinate system), a path from the position of the distal tip 443 of the lung field endoscope 40 at the current time point to the position of the region of interest on the basis of the position (coordinates of the in-vivo coordinate system) of the distal tip 443 of the lung field endoscope 40 and the position (coordinates of the in-vivo coordinate system) of the region of interest.

The bronchus of the lung indicated in the three-dimensional medical image is defined as a three-dimensional object in the in-vivo coordinate system defined in three dimensions, and the control unit 62 of the information processing device 6 specifies the path by calculating, using space information of the path inside the bronchus, a direction vector or the like from the position of the distal tip 443 of the lung field endoscope 40 to the position of the region of interest. The direction vector includes the insertion direction, an insertion amount, and an insertion speed of the lung field endoscope 40, and includes information regarding the path to the region of interest such as a lesion.

When the operation support information is output, the control unit 62 of the information processing device 6 may generate image data obtained by superimposing the operation support information on the endoscopic image or the three-dimensional medical image, and output the image data to, for example, the display unit 7. The display unit 7 displays, on the basis of the image data output from the control unit 62 of the information processing device 6, the endoscopic image or the three-dimensional medical image on which the operation support information is superimposed.

In a case where the three-dimensional medical images are reconstructed on the basis of a plurality of respiratory phases (segmented in a plurality of phase units), the control unit 62 of the information processing device 6 may perform the series of processing by switching the three-dimensional medical images according to the respiratory phase specified on the basis of the respiration synchronization signal acquired from the respiration synchronization device 91. For example, in a case where the three-dimensional medical images are reconstructed on the basis of 12 phases of respiratory phases, the control unit 62 of the information processing device 6 may perform the processing based on the three-dimensional medical images corresponding to the respective respiratory phases by performing switching 12 times, which is the number of phases of the respiratory phase, in one respiration cycle. By increasing the number of phases of the respiratory phase in reconstructing the three-dimensional medical images in this way, a synchronization timing (synchronization point) with the endoscopic image can be increased, and expansion of the operation support information to be provided to a doctor or the like can be achieved.

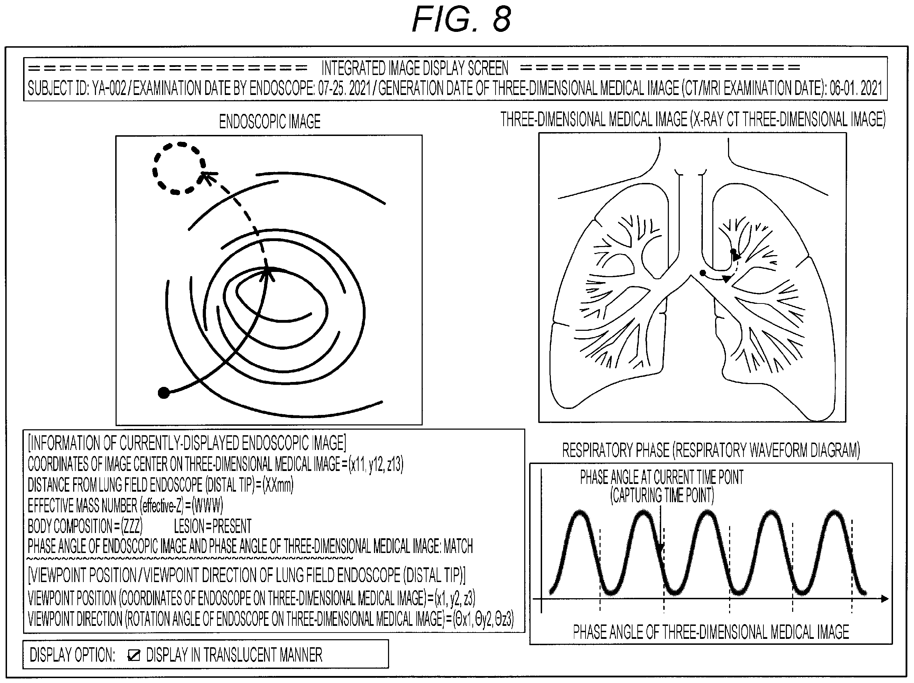

Fig. 8 is an explanatory diagram illustrating an aspect of an integrated image display screen. As described above, the control unit 62 of the information processing device 6 generates the image data including the endoscopic image or the three-dimensional medical image on which the operation support information such as the insertion direction of the lung field endoscope 40 is superimposed, and outputs the image data to the display unit 7. An integrated image display screen 71 is an example of a display screen configured by the image data, and the display unit 7 displays the integrated image display screen 71 on the basis of the image data.

The integrated image display screen 71 includes, for example, an area for displaying bibliographic matters such as a subject ID, an area for displaying an endoscopic image, an area for displaying a three-dimensional medical image, an area for displaying a respiratory waveform, and an area for displaying information regarding the currently-displayed endoscopic image, a viewpoint position (position of the distal tip 443) from which the endoscopic image is captured, and the like.

In the area for displaying the bibliographic matters such as the subject ID, bibliographic matters in data management, such as the subject ID used to specify the three-dimensional medical image corresponding to the endoscopic image, an examination date and time by the lung field endoscope 40, and a generation date of the three-dimensional medical image, are displayed.

In the area for displaying the endoscopic image, the endoscopic image captured by the lung field endoscope 40 at the current time point is displayed in real time. The operation support information such as the insertion direction of the lung field endoscope 40 is superimposed and displayed on the endoscopic image. According to setting of a display option to be described later, the endoscopic image may be displayed in a translucent manner, and an internal body site positioned on an inner side of an internal wall surface displayed in the endoscopic image may be displayed by a dotted line or the like. The internal body site displayed by the dotted line or the like in the translucent manner in this way may be, for example, a site of a lesion candidate extracted based on shape information of the internal body site specified in the three-dimensional medical image.

In the area for displaying the three-dimensional medical image, an internal body site such as a lung (bronchus) represented in the three-dimensional medical image is displayed as a three-dimensional object, and the operation support information such as the position (viewpoint position) of the distal tip 443 of the lung field endoscope 40 and the insertion direction of the lung field endoscope 40 when the position (viewpoint position) of the distal tip 443 is set as a starting point is superimposed and displayed. By dragging any site of the three-dimensional object, the three-dimensional object can be rotated. In the three-dimensional medical image, the position of the site of the lesion candidate extracted on the basis of the shape information of the internal body site specified in the three-dimensional medical image may be displayed in, for example, a highlighted state.

The endoscopic image is displayed in real time according to the capturing time point, and changes according to a lapse of time. When a phase angle of the endoscopic image at the capturing time point matches a phase angle used for reconstructing the three-dimensional medical image, the endoscopic image and the three-dimensional medical image have the same phase angle and are synchronized. The control unit 62 of the information processing device 6 may change a display mode of the distal tip 443 of the lung field endoscope 40, an arrow indicating the insertion direction, and the like to be superimposed and displayed on the endoscopic image and the three-dimensional medical image when these phase angles are the same (at the time of synchronization) and when these phase angles are different (at the time of non-synchronization). For example, when these phase angles are the same (at the time of synchronization), the distal tip 443 of the lung field endoscope 40 and the like may be superimposed and displayed in blue, and when these phase angles are different (at the time of non-synchronization), they may be superimposed and displayed in yellow. As a result, it is possible to efficiently notify an operator of the lung field endoscope 40 such as a doctor whether or not the phase angle of the endoscopic image captured at the current time point and the phase angle of the three-dimensional medical image match (are in a synchronous state).

In a case where the three-dimensional medical image is reconstructed on the basis of a plurality of respiratory phases, the control unit 62 of the information processing device 6 may switch and display the three-dimensional medical image according to the respiratory phase specified on the basis of the respiration synchronization signal acquired from the respiration synchronization device 91. As a result, in one respiration cycle, switch and display of the three-dimensional medical image are performed the number of times corresponding to the number of phases of the respiratory phase, and the three-dimensional medical image can be switched and displayed so as to correspond to a respiration cycle of a subject. By switching and displaying the three-dimensional medical image according to the respiratory phase, a synchronization timing (synchronization point) between the three-dimensional medical image and the endoscopic image can be increased, and expansion (increase in an output frequency) of the operation support information to be provided to a doctor or the like can be achieved.

In the area for displaying the respiratory waveform, a graphic drawing indicating a respiratory waveform generated on the basis of the respiration synchronization signal acquired from the respiration synchronization device 91 is displayed. The respiration synchronization device 91 is, for example, an RGSC respiration synchronization system manufactured by Varian Medical Systems, Inc., or a respiration synchronization system AZ-733VI manufactured by ANZAI MEDICAL CO., LTD. In the graphic drawing of the respiratory waveform, a horizontal axis indicates time, and a vertical axis indicates a lung volume (amount of air sucked). In the respiratory waveform, a first half of the respiration cycle indicates an inspiratory period and a second half indicates an expiratory period, and a phase angle in the respiration cycle may be defined as 0 (at a start of inspiration) to 2n (at an end of expiration). The three-dimensional medical image is reconstructed at a predetermined phase angle, and an object such as a diagram indicating the phase angle of the three-dimensional medical image is displayed in the graphic drawing of the respiratory waveform. As a result, it is possible to efficiently provide a doctor or the like with information regarding a relationship between the phase angle used for reconstructing the three-dimensional medical image and the respiration cycle.

In the graphic drawing of the respiratory waveform, an object such as an arrow indicating the phase angle of the endoscopic image displayed at the current time point may be displayed. Since the acquired endoscopic image is associated with the phase angle at the capturing time point of the endoscopic image, it is possible to efficiently provide a doctor or the like with the information regarding the relationship between the phase angle of the endoscopic image and the respiration cycle by indicating the phase angle of the endoscopic image to be displayed in the graphic drawing of the respiratory waveform. In a case where the endoscopic image is a moving image, the arrow indicating the phase angle of the endoscopic image also slides over time (moves to the right along the horizontal axis which is a time axis) along progress of the moving image (lapse of time), and is displayed.

In the area for displaying the viewpoint position where the endoscopic image is captured and the like, the position (viewpoint position) and a viewpoint direction (rotation angle) of the lung field endoscope 40 in the body at the capturing time point of the endoscopic image displayed in the area for displaying the endoscopic image are displayed. As described above, the control unit 62 (acquisition unit 621) of the information processing device 6 continuously acquires the endoscopic image, the S coordinate indicating the insertion distance of the lung field endoscope 40, and the bending history from the processor 20 for an endoscope, and continuously derives the position (viewpoint position) of the distal tip 443 of the lung field endoscope 40 on the basis of the acquired S coordinate and the like. Therefore, in the area for displaying the viewpoint position where the endoscopic image is captured and the like, the position (viewpoint position) of the distal tip 443 of the lung field endoscope 40 and the viewpoint direction (coordinates and a rotation angle in the in-vivo coordinate system) are displayed in real time following an operation of the lung field endoscope 40 by a doctor or the like.

In the area for displaying the information regarding the currently-displayed endoscopic image, for example, information regarding an internal body site (bronchus) or a pixel at an image center of the currently-displayed endoscopic image is displayed. As described above, in the three-dimensional medical image, the information regarding the internal body site (pixel) includes the effective mass number (effective-Z) or information regarding a composition (body composition) of each pixel of the three-dimensional medical image such as fat or lactic acid, which is substance determination information based on X-rays. Therefore, it is possible to display, in the area, the effective mass number (effective-Z) or the information regarding the body composition extracted from the three-dimensional medical images, on the basis of coordinates in the in-vivo coordinate system indicating the image center of the endoscopic image. In addition, presence or absence of a lesion in an internal body site included in the currently-displayed endoscopic image can also be displayed in the area by using a region of interest model (learning model) that receives the endoscopic image as an input and outputs information regarding the presence or absence of a lesion. As the region of interest model (learning model) that outputs the information regarding the presence or absence of a region of interest such as a lesion on the basis of the endoscopic image input in this way, for example, an optional object detection algorithm having a function of a segmentation network such as CNN, regions with convolutional neural network (RCNN), Fast RCNN, Faster-RCNN, single shot multibox detector (SSD), or You Only Look Once (YOLO) may be used. The phase angle of the currently-displayed endoscopic image is displayed in the graphic drawing in the area for displaying the respiratory waveform, but this region may further display whether or not the phase angle of the currently-displayed endoscopic image and the phase angle of the three-dimensional medical image match.

A display option field for setting a display option may be arranged on the integrated image display screen. The display option field is provided with a toggle switch for setting whether or not to display the endoscopic image in a translucent manner. By inputting a check in the toggle switch, on the basis of shape data or the like of an internal body site included in the three-dimensional medical image, processing of making an internal wall surface (internal wall surface of the bronchus) displayed in the endoscopic image translucent is performed, and the internal body site positioned on an inner side of the internal wall surface is displayed by a dotted line or the like. As described above, the internal body site positioned on an inner side of the internal wall surface may be, for example, a site of the region of interest such as a lesion extracted on the basis of shape information of the internal body site specified in the three-dimensional medical image.

According to the present embodiment, since the control unit 62 of the information processing device 6 specifies the position of the lung field endoscope 40 in the three-dimensional medical image synchronized with the respiratory phase, it is possible to specify, corresponding to the respiratory phase of the lung to be observed, the position of the lung field endoscope 40 in the three-dimensional medical image. In a case where an object to be captured of the three-dimensional medical image by the X-ray CT device 92 or the like is the lung, the object to be captured (lung) is accompanied by body motion due to respiration. On the other hand, by using the three-dimensional medical image synchronized with the respiratory phase so as to have a predetermined phase angle, an influence of the body motion due to respiration can be reduced and the position of the lung field endoscope 40 in the three-dimensional medical image can be specified, and accuracy of the position specification can be improved. Since the position of the lung field endoscope 40 is specified using the insertion distance (S coordinate), the bending history, and the like of the lung field endoscope inserted into the body of the subject at the time point when the endoscopic image is captured, it is possible to efficiently specify the position on the three-dimensional medical image corresponding to the insertion distance (S coordinate) and the like.

According to the present embodiment, the endoscopic image is acquired as a moving image having a frame rate of, for example, 60, and a first frame in which the phase angle in the respiratory phase is, for example, 270° (phase is 0%: exhaust) is specified in the plurality of frames. According to the synchronization signal (corresponding to the predetermined phase angle) that synchronizes the three-dimensional medical image with the endoscopic image and that is output from the respiration synchronization device 91, the endoscopic image having the same phase angle as that of the three-dimensional medical image is specified. Alternatively, processing of adding the phase angle (phase) to subsequent frames may be performed on the basis of a relationship between a capturing time point in the specified first frame and the respiration cycle. The three-dimensional medical image acquired in synchronization with the respiratory phase has a predetermined phase angle (phase) such as 270° (phase is 0%: exhaust), for example. By applying the insertion distance (S coordinate) at the time point when the frame (endoscopic image) to which the same phase angle (phase) is added is captured to the three-dimensional medical image having the predetermined phase angle (phase), it is possible to synchronize the three-dimensional medical image and the endoscopic image on the basis of the respiratory phase and to specify the position of the distal tip 443 of the lung field endoscope 40 in the three-dimensional medical image.

According to the present embodiment, according to the present embodiment, the control unit 62 of the information processing device 6 segments the plurality of acquired three-dimensional medical images in a plurality of phase units including four segments of, for example, 90° (inspiration: 50%), 180° (inspiration: 100%), 270° (exhaust (expiration): 50%), and 360° (exhaust (expiration): 100%), and specifies the position of the lung field endoscope 40 for each segmented three-dimensional medical image. As a result, sampling corresponding to the number of segmentations can be performed in one cycle in the respiratory phase, and a synchronization frequency of the three-dimensional medical image with the endoscopic image can be improved. Since the three-dimensional medical images segmented in the plurality of phase units are switched and output according to the respiratory phase, it is possible to efficiently provide an operator of the lung field endoscope 40 such as a doctor with information corresponding to body motion due to respiration by the lung of the subject.

According to the present embodiment, the operation support information includes the insertion direction, the insertion amount, and the insertion speed of the lung field endoscope 40, and the information regarding target point coordinates (coordinates of the region of interest such as a lesion) indicating an insertion destination. Thus, it is possible to provide an operator of the lung field endoscope 40 such as a doctor with useful information, and to contribute to diagnosis support for the doctor or the like.

### (Second Embodiment)

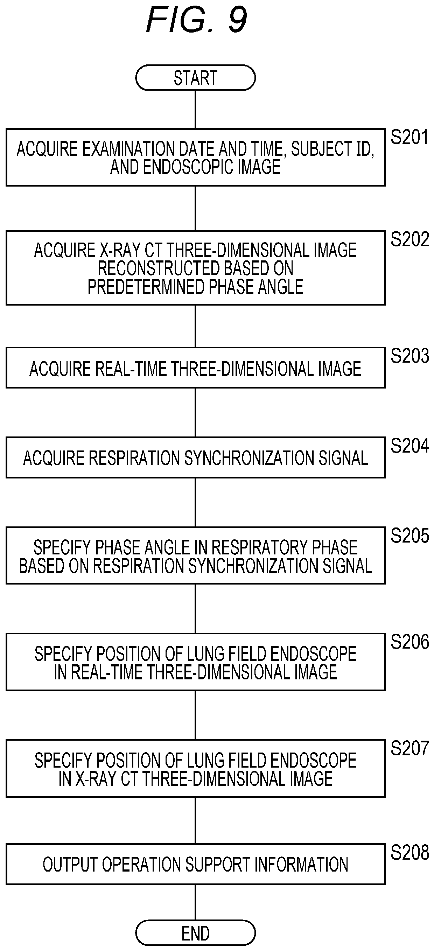

Fig. 9 is a flowchart illustrating an example of a processing procedure performed by a control unit 62 of an information processing device 6 according to a second embodiment (real-time three-dimensional image). Similarly to the first embodiment, the control unit 62 of the information processing device 6 starts processing of the flowchart on the basis of, for example, contents input from an input unit 8 connected to the device.

The control unit 62 of the information processing device 6 acquires an examination date and time, a subject ID, and an endoscopic image, which are output from a processor 20 for an endoscope (S201). The control unit 62 of the information processing device 6 acquires the examination date and time, the subject ID, and the endoscopic image, which are output from the processor 20 for an endoscope, similarly to the processing S101 of the first embodiment. In the present embodiment, the control unit 62 of the information processing device 6 does not need to acquire an insertion distance (S coordinate) and a bending history.

The control unit 62 of the information processing device 6 acquires, from an X-ray CT device 92, an X-ray CT three-dimensional image reconstructed on the basis of a predetermined phase angle (S202). In the present embodiment, a three-dimensional medical image acquired by the control unit 62 of the information processing device 6 includes an X-ray CT three-dimensional image captured by X-ray CT and a real-time three-dimensional image captured by a CT fluoroscopy function to be described later. The X-ray CT three-dimensional image corresponds to the three-dimensional medical image described in the first embodiment, and is an image reconstructed on the basis of a CT image (tomographic image data) based on a result of examination by the X-ray CT device 92 or the like performed on the same subject before examination of a lung (bronchus) (capturing of the endoscopic image) by a lung field endoscope 40. The processing of S202 is performed similarly to the processing of S102 of the first embodiment, and the X-ray CT three-dimensional image reconstructed on the basis of the predetermined phase angle is acquired from the X-ray CT device 92.

The control unit 62 of the information processing device 6 acquires the real-time three-dimensional image from the X-ray CT device 92 (S203). The X-ray CT device 92 is, for example, a high-speed X-ray CT device, and is mounted with the CT fluoroscopy function. The control unit 62 of the information processing device 6 acquires the real-time three-dimensional image captured by the CT fluoroscopy function from the X-ray CT device 92. By using the CT fluoroscopy function mounted in the X-ray CT device 92 (high-speed X-ray CT device), it is possible to continuously reconstruct a three-dimensional image in real time in units of one second or the like and capture (reconstruct) the real-time three-dimensional image.

The capturing and the reconstruction of the real-time three-dimensional image by the CT fluoroscopy function are performed simultaneously and in parallel with the capturing (acquisition) of the endoscopic image, and the reconstruction of the real-time three-dimensional image and the capturing (acquisition) of the endoscopic image are continuously processed on the same time axis. Therefore, the real-time three-dimensional image includes the lung field endoscope 40 inserted into the bronchus of the lung.

The control unit 62 of the information processing device 6 acquires, from a respiration synchronization device 91, a signal (respiration synchronization signal) corresponding to a cycle of respiration (respiration cycle) (S204). The control unit 62 of the information processing device 6 specifies a phase angle in a respiratory phase on the basis of the respiration synchronization signal (S205). The control unit 62 of the information processing device 6 performs the processing of S204 and S205 similarly to the processing of S103 and S104 of the first embodiment. The control unit 62 of the information processing device 6 may associate the specified phase angle with the endoscopic image and the real-time three-dimensional image.

The control unit 62 of the information processing device 6 specifies a position of the lung field endoscope 40 in the real-time three-dimensional image (S206). The real-time three-dimensional image is also defined by an in-vivo coordinate system indicated in three dimensions, similarly to the X-ray CT three-dimensional image. The real-time three-dimensional image reconstructed simultaneously in parallel with the capturing of the endoscopic image includes the lung field endoscope 40 obtained by capturing the endoscopic image. The control unit 62 of the information processing device 6 performs edge detection, pattern detection, or the like on the real-time three-dimensional image to specify a distal tip 443 of the lung field endoscope 40, and calculates a position (coordinates of the in-vivo coordinate system) of the distal tip 443 to specify the position of the lung field endoscope 40 (distal tip 443) in the real-time three-dimensional image.

The control unit 62 of the information processing device 6 specifies the position of the lung field endoscope 40 in the X-ray CT three-dimensional image by performing matching processing between the X-ray CT three-dimensional image and the real-time three-dimensional image (S207). When the matching processing between the X-ray CT three-dimensional image and the real-time three-dimensional image is performed, the control unit 62 of the information processing device 6 performs, for example, comparison processing using cosine similarity on both images, and determines that these images match in a case where a matching degree (similarity) of the images as a comparison result is equal to or greater than a predetermined value. Alternatively, in the matching processing between the X-ray CT three-dimensional image and the real-time three-dimensional image, it may be determined that these images match using an image matching degree model that outputs (estimates), in a case where the X-ray CT three-dimensional image and the real-time three-dimensional image are input, a matching degree (similarity) of these images. That is, in a case where the matching degree (similarity) output by the image matching degree model is equal to or greater than a predetermined value, it may be determined that these images match. Alternatively, the matching processing between the X-ray CT three-dimensional image and the real-time three-dimensional image may use aligning processing between coordinate systems (in-vivo coordinate systems) in these three-dimensional images. By performing the aligning processing between the coordinate systems, the position (coordinates) of the lung field endoscope 40 (distal tip 443) specified in the real-time three-dimensional image may be converted into a position (coordinates) in the coordinate system in the X-ray CT three-dimensional image, and the position (distal tip 443) of the lung field endoscope 40 in the X-ray CT three-dimensional image may be specified.

The control unit 62 of the information processing device 6 may superimpose and output the specified position of the lung field endoscope 40 (distal tip 443) on the X-ray CT three-dimensional image having higher resolution than that of the real-time three-dimensional image. Since the X-ray CT three-dimensional image is synchronized with the respiratory phase, an influence of body motion due to respiration can be reduced and the position of the lung field endoscope 40 in the three-dimensional medical image can be specified, and accuracy of the position specification can be improved.

The control unit 62 of the information processing device 6 outputs operation support information on the basis of the derived position of the lung field endoscope 40 and a position of a region of interest included in the three-dimensional medical image (X-ray CT three-dimensional image, real-time three-dimensional image) (S208). The control unit 62 of the information processing device 6 performs the processing of S208 similarly to the processing of S106 of the first embodiment.

According to the present embodiment, the three-dimensional medical image includes the X-ray CT three-dimensional image captured by the X-ray CT before the examination by the lung field endoscope 40 and the real-time three-dimensional image captured by the CT fluoroscopy function simultaneously with the examination by the lung field endoscope 40. For example, by using the CT fluoroscopy function mounted in the high-speed X-ray CT device, it is possible to continuously reconstruct a three-dimensional image in real time in units of one second or the like and capture (reconstruct) the real-time three-dimensional image. Since the capturing of the real-time three-dimensional image is performed simultaneously with the examination by the lung field endoscope 40, that is, the capturing of the bronchus of the lung by the lung field endoscope 40, the distal tip 443 of the lung field endoscope 40 is included in the real-time three-dimensional image. As a result, the position of the lung field endoscope 40 (distal tip 443) in the lung (bronchus) indicated by the real-time three-dimensional image can be specified on the basis of the coordinates of the lung field endoscope 40 (distal tip 443) in the image coordinate system of the real-time three-dimensional image. When the position of the lung field endoscope 40 is specified on the basis of the lung field endoscope 40 included in the real-time three-dimensional image, the matching processing between the X-ray CT three-dimensional image and the real-time three-dimensional image may be performed to specify the position of the lung field endoscope 40 in the X-ray CT three-dimensional image.

The control unit 62 of the information processing device 6 superimposes and outputs the specified position of the lung field endoscope 40 (distal tip 443) on the real-time three-dimensional image and the X-ray CT three-dimensional image having higher resolution than that of the real-time three-dimensional image. Since the X-ray CT three-dimensional image is synchronized with the respiratory phase, an influence of body motion due to respiration can be reduced and the position of the lung field endoscope 40 in the X-ray CT three-dimensional image can be specified, and accuracy of the position specification can be improved.

According to the present embodiment, the X-ray CT three-dimensional image is captured before the real-time three-dimensional image and the examination by the lung field endoscope 40, and the X-ray CT three-dimensional image includes the entire lung of the subject. Therefore, in a case where there is a region of interest (ROI) such as a lesion such as a tumor or a lesion candidate in the lung, the region of interest is included in the X-ray CT three-dimensional image. In the X-ray CT three-dimensional image synchronized with the respiratory phase, since the position (current point) of the lung field endoscope 40 (distal tip 443) at a current time point (at a capturing time point of the endoscope) is specified, it is possible to specify a path from the current point to a point where the region of interest is positioned using the X-ray CT three-dimensional image and derive the operation support information for inserting the lung field endoscope 40 along the path.

### (Third Embodiment)

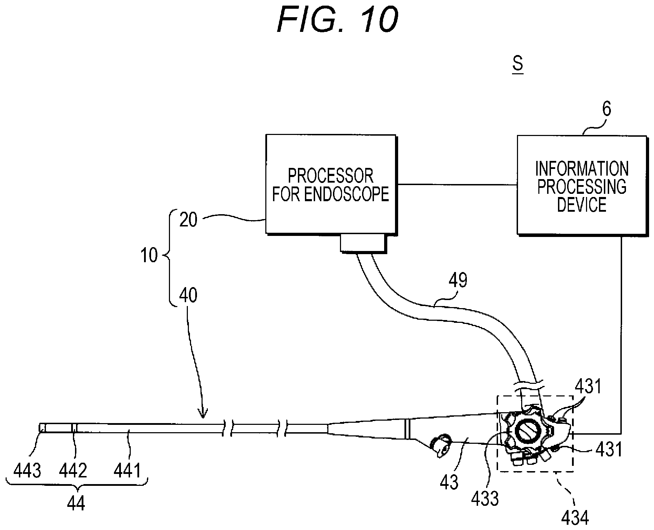

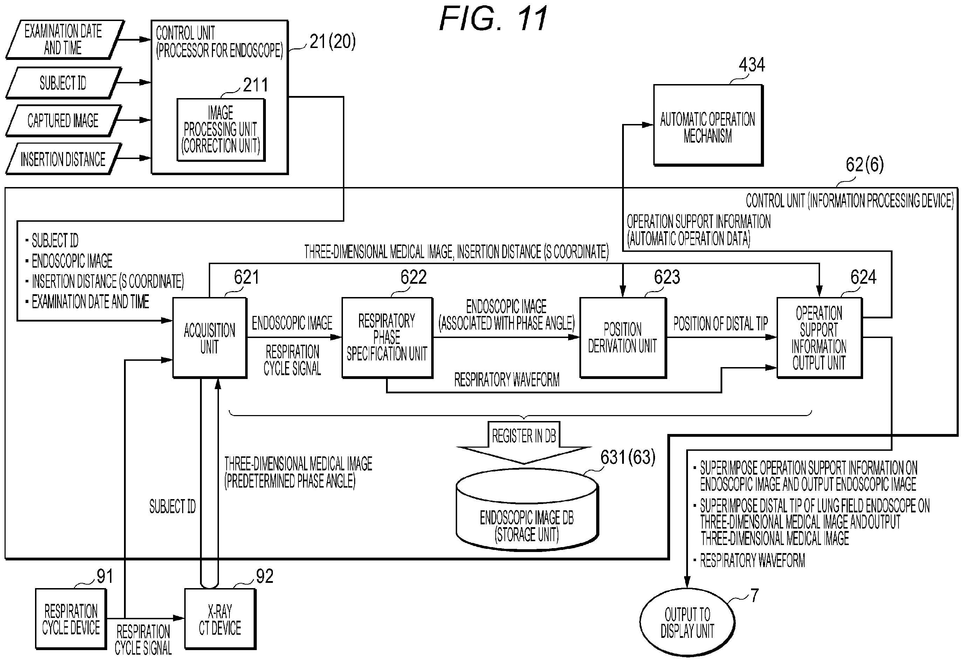

Fig. 10 is a schematic diagram illustrating an outline of a diagnosis support system according to a third embodiment (automatic operation mechanism 434). Fig. 11 is a functional block diagram exemplifying functional units included in a control unit of an information processing device 6. A lung field endoscope 40 included in a diagnosis support system S of the third embodiment includes the automatic operation mechanism 434 that automatically operates a control button 431 and a bending knob 433 on the basis of operation support information output from a control unit 62 (operation support information output unit 624) of the information processing device 6.

The automatic operation mechanism 434 is communicably connected to the information processing device 6, and acquires (receives) the operation support information output (transmitted) from the information processing device 6. The automatic operation mechanism 434 includes, for example, a microcomputer (not illustrated) that generates an on/off signal or a pulse signal for the control button 431 or the bending knob 433 from the acquired operation support information, and a motor/cam mechanism (not illustrated) that operates or drives the control button 431 or the bending knob 433 on the basis of these signals output from the microcomputer. On the basis of the operation support information output from the information processing device 6 in this way, the automatic operation mechanism 434, the control button 431, the bending knob 433, and the like cooperate with each other, and perform an automatic operation such as automatic insertion of the lung field endoscope 40 into a body of a subject according to the operation support information.

Since the operation support information includes information regarding a path from a position of a distal tip 443 of the lung field endoscope 40 at a current time point to a position of a region of interest such as a lesion, it is possible to cause the distal tip 443 of the lung field endoscope 40 to reach the position of the region of interest by performing the automatic operation according to the operation support information. Since the operation support information is derived when phase angles of an endoscopic image and a medical image for three dimensions match, it is possible to reduce an influence of body motion due to respiration in the lung (body motion) and perform the automatic operation such as the automatic insertion of the lung field endoscope 40.

The operation support information acquired by the automatic operation mechanism 434 from the control unit 62 (operation support information output unit 624) of the information processing device 6 is not limited to the operation support information such as insertion and bending of the insertion portion 44. For example, the insertion portion 44 of the lung field endoscope 40 may be provided with an air injection portion (not illustrated) or a hand portion (not illustrated), and the operation support information may include information regarding an operation such as injection of air by the air injection portion or extraction (sampling) of a lesion site by the hand portion. That is, the operation support information output unit 624 generates the information regarding the operation of the air injection portion or the hand portion on the basis of shape information and distance image information of an internal body site (bronchus) specified in the acquired medical image for three dimensions, includes the information in the operation support information, and outputs the operation support information to the automatic operation mechanism 434. The automatic operation mechanism 434 may automatically operate the air injection portion or the hand portion on the basis of the acquired operation support information. Note that the information regarding the operation of the air injection portion or the hand portion included in the operation support information may be superimposed and displayed on the endoscopic image and the like on an integrated image display screen 71.

According to the present embodiment, on the basis of the position of the lung field endoscope 40 specified in the three-dimensional medical image synchronized with a respiratory phase, it is possible to efficiently output the operation support information regarding the operation of the lung field endoscope 40, and the automatic operation mechanism 434 automatically operates the lung field endoscope 40 according to the operation support information output from the information processing device 6. Therefore, it is possible to provide an operator who operates the lung field endoscope 40 such as a doctor with the diagnosis support system S that efficiently supports the operation.

The embodiments disclosed herein are considered to be illustrative in all respects and not restrictive. The technical features described in the embodiments can be combined with each other, and the scope of the present invention is intended to include all modifications within the scope of the claims and the scope equivalent to the claims.

### Reference Signs List

- S: Diagnosis support system
- 10: Endoscope device
- 15: Keyboard
- 16: Storage shelf
- 20: Processor for an endoscope
- 21: Control unit
- 211: Image processing unit
- 22: Main storage device
- 23: Auxiliary storage device
- 24: Communication unit
- 25: Touch panel
- 26: Display device I/F
- 27: Input device I/F
- 28: Reading unit
- 31: Endoscope connector
- 311: Electrical connector
- 312: Optical connector
- 33: Light source
- 40: Lung field endoscope
- 43: Operation unit
- 431: Control button
- 433: Bending knob
- 434: Automatic operation mechanism
- 44: Insertion portion (flexible tube)
- 441: Soft portion
- 442: Bending section
- 443: Distal tip
- 444: Image capturing unit
- 445: Image sensor
- 446: Image capturing light source
- 45: Bend preventing portion
- 48: Scope connector
- 49: Universal cord
- 50: Display device
- 6: Information processing device
- 61: Communication unit
- 62: Control unit
- 621: Acquisition unit
- 622: Respiratory phase specification unit
- 623: Position derivation unit
- 624: Operation support information output unit
- 63: Storage unit
- 631: Endoscopic image DB
- 632: Recording medium
- P: Program (program product)
- 64: Input/output I/F
- 7: Display unit
- 8: Input unit
- 91: Respiration synchronization device
- 92: X-ray CT device

## Claims

1. A program for causing a computer to execute processing comprising:
acquiring information regarding a respiratory phase of a subject;
acquiring a three-dimensional medical image obtained by capturing an inside of a body of the subject in synchronization with the respiratory phase;
acquiring an endoscopic image of the subject from a lung field endoscope;
specifying a position of the lung field endoscope in the three-dimensional medical image synchronized with the respiratory phase; and
outputting the specified position of the lung field endoscope in association with the three-dimensional medical image.

2. The program according to claim 1, wherein
the processing of specifying the position of the lung field endoscope includes processing of:
acquiring, in association with the respiratory phase, information regarding an insertion distance of the lung field endoscope inserted into the body of the subject at a time point when the endoscopic image is captured; and
specifying the position of the lung field endoscope in the three-dimensional medical image synchronized with the respiratory phase on the basis of the information regarding the insertion distance associated with the respiratory phase.

3. The program according to claim 1 or 2, wherein
the three-dimensional medical image is reconstructed at a predetermined phase angle in the respiratory phase, and
information regarding a correspondence situation between a phase angle of the acquired endoscopic image at the capturing time point and a phase angle of the three-dimensional medical image is output.

4. The program according to claim 3, wherein
a plurality of the acquired three-dimensional medical images is segmented in a plurality of phase units in the respiratory phase, and
the position of the lung field endoscope is specified for each of the segmented three-dimensional medical images.

5. The program according to claim 4, wherein
the three-dimensional medical images segmented in the plurality of phase units are switched and output according to the respiratory phase.

6. The program according to any one of claims 1 to 5, wherein
the three-dimensional medical image includes a real-time three-dimensional image captured using a CT fluoroscopy function in parallel with capturing of the endoscopic image by the lung field endoscope, and
the position of the lung field endoscope is specified on the basis of the lung field endoscope included in the real-time three-dimensional image.

7. The program according to claim 6, wherein
the three-dimensional medical image includes an X-ray CT three-dimensional image captured by an X-ray CT device and the real-time three-dimensional image, and
matching processing between the X-ray CT three-dimensional image and the real-time three-dimensional image is performed to specify the position of the lung field endoscope in the X-ray CT three-dimensional image on the basis of the lung field endoscope included in the real-time three-dimensional image.

8. The program according to any one of claims 1 to 7, wherein
operation support information regarding an operation of the lung field endoscope is output on the basis of the three-dimensional medical image in which the position of the lung field endoscope is specified.

9. The program according to claim 8, wherein
the operation support information includes information regarding a path to a region of interest derived on the basis of the three-dimensional medical image.

10. The program according to claim 8 or 9, wherein
the operation support information is output to an automatic operation mechanism that performs an automatic operation of the lung field endoscope.

11. An information processing method for causing a computer to execute processing comprising:
acquiring information regarding a respiratory phase of a subject;
acquiring a three-dimensional medical image obtained by capturing an inside of a body of the subject in synchronization with the respiratory phase;
acquiring an endoscopic image of the subject from a lung field endoscope;
specifying a position of the lung field endoscope in the three-dimensional medical image synchronized with the respiratory phase; and
outputting the specified position of the lung field endoscope in association with the three-dimensional medical image.

12. An information processing device comprising:
a phase information acquisition unit that acquires information regarding a respiratory phase of a subject;
a three-dimensional medical image acquisition unit that acquires a three-dimensional medical image obtained by capturing an inside of a body of the subject in synchronization with the respiratory phase;
an endoscopic image acquisition unit that acquires an endoscopic image of the subject from a lung field endoscope;
a position specification unit that specifies a position of the lung field endoscope in the three-dimensional medical image synchronized with the respiratory phase; and
an output unit that outputs the specified position of the lung field endoscope in association with the three-dimensional medical image.

13. A diagnosis support system comprising:
a lung field endoscope;
an automatic operation mechanism that automatically operates the lung field endoscope; and
a control unit that controls the automatic operation mechanism, wherein
the control unit
acquires information regarding a respiratory phase of a subject,
acquires a three-dimensional medical image obtained by capturing an inside of a body of the subject in synchronization with the respiratory phase,
acquires an endoscopic image of the subject from the lung field endoscope,
specifies a position of the lung field endoscope in the three-dimensional medical image synchronized with the respiratory phase, and
outputs operation support information regarding an operation of the lung field endoscope to the automatic operation mechanism on the basis of the three-dimensional medical image in which the position of the lung field endoscope is specified.
